# EUROPEAN PATENT SPECIFICATION

(11) **EP 2 113 201 B1**
(45) Date of publication and mention of the grant of the patent: **17.07.2013**
(21) Application number: 09157872.4
(22) Date of filing: 14.04.2009
(51) Int. Cl.: A61B 8/08, G10K 11/00, G10K 11/35, A61B 8/12, G01S 15/89

(54) **Moveable ultrasound element for use in medical diagnostic equipment**
Bewegliches Ultraschallelement zur Verwendung in medizinischen Diagnosevorrichtungen
Élément ultrasonique mobile pour une utilisation dans un équipement de diagnostic médical

(30) Priority: 14.04.2008 US 124064 P; 13.04.2009 US 422766
(43) Date of publication of application: 04.11.2009
(60) Divisional application: 12195327.7
(73) Proprietor: iVu Imaging Corporation, Grapevine TX 76051 (US)
(72) Inventor: Stribling, Mark L., Johnson City, TN 37601 (US)
(74) Representative: Johnstone, Douglas Ian

(56) References cited:
- WO-A2-01/45550
- US-A- 4 231 373
- US-A- 4 957 099
- US-A1- 2005 228 283

## Description

The present invention relates to ultrasound equipment, and more particularly, to ultrasound equipment useable in a medical diagnostic application, such as in the examination of tissue such as breast and/or prostate tissue for determining the presence or absence of abnormal tissues, such as tumors associated with cancer.

Ultrasound machines have long been used in medical diagnostic applications. For example, ultrasound machines are used to obtain real-time images of fetus in-utero, and are also used to detect certain anomalies in tissues, such as the presence or absence of cancerous tissue that may exist in breasts for breast cancer detection; in prostates for prostate cancer detection and the like. Ultrasound machines are also used for detecting cancers located in the ovaries, thyroid, abdomen, liver, spleen, pancreas and kidneys.

An ultrasound machine operates through the use of sound waves that are transmitted and received in a manner similar to "radar" that can be used to create an image of a tissue of interest. The main component of an ultrasound machine is the transducer probe. The transducer probe creates and transmits the sound waves and receives the echoes by employing the piezoelectric effect. The piezoelectric effect operates through the use of one or more quartz crystals that are often referred to as piezoelectric crystals. When an electric charge is applied to these piezoelectric crystals, the crystals rapidly change shape, thereby producing sound waves that travel outwardly. When returning sound waves strike the crystals, they also change shape, and emit electric currents.

Along with the transducer probe, most ultrasound machines include a central processing unit that performs the calculations necessary to accomplish the tasks desired of the crystal. The central processing unit also contains the electrical power supplies for itself and the transducer probe, and transducer pulse controls that change the amplitude, frequency and duration of the pulse emitted from the transducer probe. An ultrasound machine may also include a display that displays the image from the ultrasound data processed by the CPU and a keyboard or pointing device (e.g. a mouse) that enables the user to input data and take measurements from the display. A data storage device, such as a hard drive, flash drive, etc., is usually also included for storing the acquired images.

Although existing ultrasound machines are highly useful diagnostic tools, room for improvement still exists to overcome certain deficiencies. Some of these deficiencies are illustrated with respect to the problems that deal with using ultrasound machines to detect abnormalities and pathologies of the breast, such as breast cancer.

The currently existing ultrasound diagnosis technology is to provide an ultrasound probe that comprises a sensor wand or handle. Within the sensor wand or handle is contained an array of ultrasound transducers (the piezoelectric crystals). These transducers are fixedly positioned with respect to the handle. In order to capture an ultrasound image of a tissue, the ultrasound wand is moved manually by the user around the subject tissue. The wand that is typically used for detecting breast cancer comprises a 128 element linear array. In principal, this ultrasound wand operates analogously to a hand-held scanner of the type used to scan documents, as the wand comprises a sort of linear array (line) of transducers.

To perform an ultrasonic breast exam, the hand-held transducer is moved by the medical practitioner so that the linear array of transducers is moved over the breast. At the same time that the transducer-containing wand is being moved over the breast, pictures are being captured and shown on a screen (display). The technician continues to move the wand and watch the display until the technician finds something in the image that the technician believes to be an abnormal (e.g. a cancerous) tissue. In breast cancer, a cancerous tissue would tend to show up as a white calcified cell or a dark area. When the technician finds a spot or other areas of interest, the technician holds the wand stationary and captures an ultrasonic "picture" from that spot or area of interest.

After capturing an image of the area of interest, the technician may continue the examination of the remainder of the breast to determine whether any other areas of interest exist. Alternately, the technician may just try to get a few more pictures of the spot or area of interest where the technician found the cancerous tissue.

The persons who usually perform this function are sonogram ("ultrasound") technicians. Sonogram technicians are similar to x-ray technicians, as their primary job is to operate the diagnostic device to obtain those images (radiographic or sonographic) requested by the physician of the area or tissue of interest. Once the image is obtained, the image is given to an interpreting physician who then reads the pictures and makes a determination or diagnosis of whether the pictures represent the existence of a pathological condition such as cancer, a tumor or some other abnormality. In the plurality of cases, the "interpreting physician" will be a radiologist. However, physicians other than radiologists are often the interpreting physician. It should be understood that the use of the term "radiologist" in this application should not to be interpreted to exclude other physicians and medical practitioners.

Currently, breast cancer is usually diagnosed by employing a three-stage screening/detection process. The first stage involves a mammogram that uses x-rays for detection of pathologies. In an x-ray type mammogram, the breast is usually squeezed between a pair of plates and an x-ray is taken. The x-ray is then read by the radiologist. If the radiologist detects the presence of either a white spot or a dark area, an ultrasound is then ordered as the second stage in the screening.

Although mammograms have been used for the last thirty years as a primary screening tool to determine the existence of breast cancer, mammograms do have certain drawbacks. A first drawback to the use of mammography is that the procedure, wherein a breast is squeezed between two plates is often painful, or uncomfortable to the user. A second problem associated with mammography is that the use of mammography entails exposing the patient to radiation. Although technological improvements have reduced the amount of radiation to which the patient is exposed, mammography still requires the use of radiation.

A more pressing problem with mammography is that its use as a diagnostic tool is rather limited, due to the limitations of the procedure. One limitation in the procedure is the mammography is only capable of providing a two-dimensional view of the breast.

Although mammography is very useful in determining whether suspicious areas exist, the presence of a suspicious area usually requires a further procedure performed by a different technique to confirm or deny the existence of cancerous tissue.

Another difficulty with mammography is that certain factors and/or conditions exist that interfere with the ability of a mammogram to provide a reliable screening because these conditions interfere with a mammogram. One example of such a condition is the presence of talcum powder, deodorants, creams or lotions that are applied under the arms or on the breasts of the patient.

Breast implants also interfere with a mammogram, as they may prevent complete visualization of the breast. Further, previous breast surgery can also interfere with an interpretation of a mammogram. Additionally, breast changes that may occur during menstruation interfere with a mammogram. Another condition that interferes with mammograms involve the existence of dense breast tissue, that often is characteristic of young women, and especially women under 30.

In addition to screening mammograms, diagnostic mammograms also exist. A diagnostic mammogram is an x-ray exam of the breast in a woman who either has a breast problem, such as a lump, nipple discharge, or has something abnormal found during a screening mammogram.

A diagnostic mammogram differs from a screening mammogram, as more pictures are taken to better and more carefully study the area of concern. Often, special pictures are enlarged to make small areas of suspicious breast tissue larger and easier to evaluate. Additionally, other types of x-ray pictures can be taken, the nature of which depends upon the type of problem and the location of the problem in the breast.

For a good discussion of other breast cancer detection techniques, including the use of mammograms, ultrasounds, MRIs and other techniques for imaging the breast, see, American Cancer Society "Mammograms and Other Breast Imaging Procedures", www.cancer.org, 2005.

After a mammogram has been performed, an ultrasound is often used as the second stage of the screening. If the ultrasound comes back positive, the third step in the process is to perform a tissue biopsy. Although this method is the current state-of-the-art, there are several deficiencies with it.

Another example of detection through ultrasound relates to the manner in which prostate cancer is detected. In a prostate cancer ultrasound procedure, a probe is inserted rectally. The probe generally comprises a cigar tube-shaped having a rounded distal end for facilitating insertion. Along the outer surface of the "tube" is a linear array of ultrasound transducers. The cigar-shaped wand is inserted rectally until it is positioned adjacent to the prostate. When placed in this position, the ultrasound array is preferably positioned so that it is facing the wall of the large intestines that is next to the prostate. The linear ultrasound array is then employed to take a linear array of ultrasound pictures while the doctor or sonogram technician manipulates the wand in order to look at various parts of the prostate. This manipulation occurs by rotating the cigar tube about its axis so that the technician can capture pictures of various parts of the prostate.

From this point on, the procedure is similar to a breast exam procedure as the technician rotates the wand to capture views of the entire prostate until the technician has either viewed the entire prostrate (and found nothing abnormal) or else the technician has located an area of tissue having a cancerous appearance or that is otherwise the subject of interest, be it a cyst, cancer cells, etc. The technician then captures pictures of the subject of interest. The captured pictures are then given to an interpreting physician who "reads" the ultrasonic picture to make a diagnosis from the picture.

Several problems exist with this method of performing an ultrasound procedure. Many of these problems spring from the fact that the pictures or images captured by the ultrasound are difficult to interpret and do not often present a very good picture of what is going on in the tissue or organ of interest.

In traditional ultrasound, the images that are acquired from the linear transducers are two dimensional images that take a picture of a paper thin "section". The problem with such an image is that it does not place the image in context with what the ultrasound is actually "looking at" visa-vis the tissue that is next to it. This two dimensional, thin section technique may lead the technician or radiologist to miss abnormal anatomical features completely if the ultrasound "picture" is taken so that it is looking at the right area and at the right angle. When something abnormal is found, the abnormal tissue may be shown in the picture at an angle that presents a view that does not portray the abnormality well.

One way to understand how mis-characterizations exist is to imagine that you are using such a linear array of transducers to look at a soccer ball. Because of the nature of the linear array, you are only permitted, at any one time, to look at a thin slice of the soccer ball. Hence, if you are looking at a thin slice on the left side of the soccer ball, you may miss an abnormality on the right side of the soccer ball. This inability of current ultrasounds to obtain an integrated picture of the entire organ of interest is one of the major deficiencies of current ultrasound devices.

One of the problems with known current ultrasound systems is that it is very difficult, if not impossible, to obtain a three-dimensional image. Even though a three-dimensional image could be obtained in theory by using a moving probe, the movement of the probe manually (which is currently being done) does not provide precise enough movements to enable even a sophisticated software package to reliably stitch together the photographs into a three-dimensional image.

WO 01/45550 discloses an ultrasound device comprising a curved array of transducer elements wherein each element is mounted on a shaft. Actuators are arranged to rotate the shafts and hence rotate the transducer elements. During ultrasound imaging, each transducer element is used to scan its field of view and form an image frame.

In accordance with the present invention, an ultrasound device is provided for diagnosing pathologies in a tissue of interest. The ultrasound device comprises a platform member and an array of at least two ultrasonic elements coupled to the platform member. Each of the at least two elements includes a body portion and a piezoelectric crystal member. The piezoelectric crystal member has a first axis and a second axis. A first mover is provided that is capable of moving the piezoelectric crystal member about a first axis of the crystal. A second mover is provided that is capable of moving the piezoelectric crystal about a second axis of the crystal. A controller is provided for causing the first mover and second mover to move the piezoelectric crystal about the respective first and second axes for enabling the ultrasound device to capture a plurality of images at different positions of rotation about the first and second axes.

Preferably, the first and second axes of the piezoelectric crystal comprise respectively an x-axis and a y-axis, wherein the mover is capable of moving the piezoelectric crystal about the x-axis, and a second mover is capable of moving the piezoelectric crystal about the y-axis.

Imaging software is preferably provided for correlating the plurality of images to produce at least one of a thin section image and a three-dimensional image. The imaging software should be capable of producing a plurality of thin section images that show parallel planes of the tissue of interest, and/or show views of a particular tissue of interest from different aspects and angles.

The first mover preferably comprises a band that is comprised of a material capable of expanding and contracting under the influence of energy applied to the band, wherein the expansion and contraction of the material of the band moves the piezoelectric crystal about the first axis. Most preferably, the band is comprised of a metal material, and includes a first end coupled to the body portion of the ultrasonic element, and a second end coupled to a first edge portion of the piezoelectric crystal. The second mover can be constructed similarly to the first mover.

The device also should include a tissue receiving member for receiving a tissue member, so that images can be taken of the tissue of interest. The platform is fixedly coupled to the tissue receiving member, and the body portions of at least two elements are fixedly coupled to the platform member. The piezoelectric crystal members are movable relative to the platform in the body portion.

In one embodiment, the tissue receiving member comprises a cup that itself comprises the platform to which the array of at least two ultrasonic elements are coupled. The array of at least two ultrasonic elements preferably comprises at least four arrays of at least two ultrasonic elements that are circumferentially arrayed around the cup to enable the ultrasonic elements to capture images fully around an axis of the tissue of interest.

Embodiments of the present invention will now be described by way of example with reference to the accompanying drawings, in which:-

Fig. 1 is a schematic view of an individual motorized ultrasound element;

Fig. 2 is a schematic representation of the movement of the ultrasound by the motor in two of the degrees of freedom, each way about the Y axis;

Figs. 3A, 3B and 3C are schematic representations of movement of the piezoelectric crystal by the motor showing movement around the X axis;

Fig. 4 is a schematic side view of an ultrasound array that is used with a breast specific apparatus for performing ultrasound on a breast;

Fig. 5 is a bottom view of the ultrasound probe apparatus useable in examining a breast;

Fig. 6 is a schematic top view of a probe useable for an internal body part, such as a prostate;

Fig. 6A is a schematic side view of the probe of Fig. 6;

Fig. 7 is a schematic representation to illustrate the data points taken by the moveable ultrasound probe of the present invention;

Fig. 8 is a first embodiment cylindrically shaped ultrasound probe element of the present invention;

Fig. 9 is a second embodiment, relatively longer, rectangularly cuboid ultrasound probe element; and

Fig. 10 is a third embodiment that comprises a relatively shortened rectangular cuboid probe element;

Fig. 11 shows a multi-element array of cylindrical elements that are all disposed on a common platform, but each of which is individually moveable with respect to the remainder of the elements;

Fig. 12 is a diagrammatic view of the primary components of the ultrasound device of the present invention;

Fig. 12A is a schematic representation of the software functionalities used in connection with the present invention; and

Fig. 13 is a schematic view of a body part illustrating exemplary planes through which an ultrasound picture can be taken.

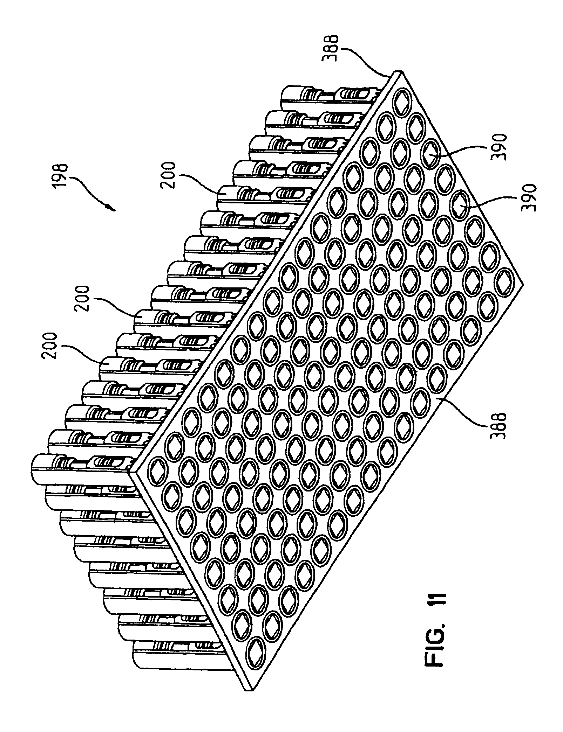

The present invention comprises an ultrasound device and in particular an ultrasound device particularly well suited for use in medical diagnostic applications. The ultrasound device 198 is comprised of a plurality of individual ultrasound elements 200 (Fig. 11). The ultrasound elements 200 are placed in a fixture 388, and include crystals 390 that are individually moveable with respect to the fixture 388.

One advantage of the present invention is that each of the individual elements 200 are individually moveable on an individual basis.

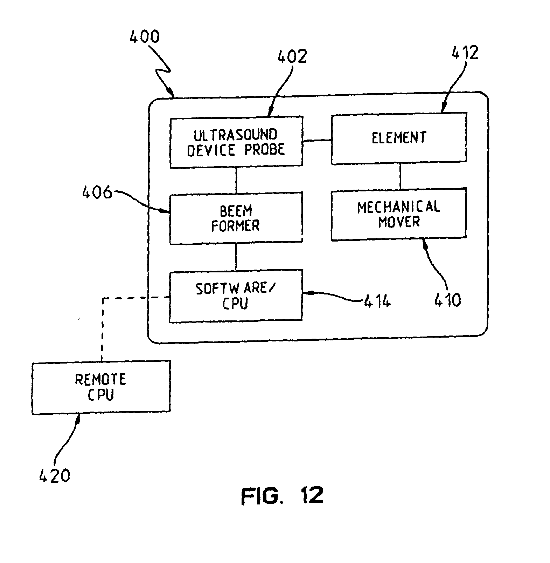

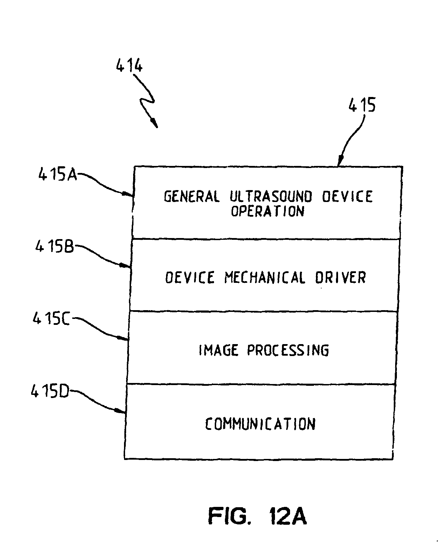

In Fig. 12, ultrasound device system 400 is shown schematically. The ultrasound device 400 includes an ultrasound probe 402 that is designed to employ ultrasonic waves to enable a user to perform medical diagnoses. The ultrasound device 400 includes a digital beam former 406 that controls the beam produced by the ultrasound device 400. A mechanical driver 410 is provided for each of the individual ultrasound elements 412 that contain a piezoelectric crystal for generating and receiving ultrasound sound waves. Software for controlling the operation of the device 400, along with a CPU 414 is also provided. Among the elements or modules of the software is a mechanical driver module (or a mechanical module functionality routine within the software).

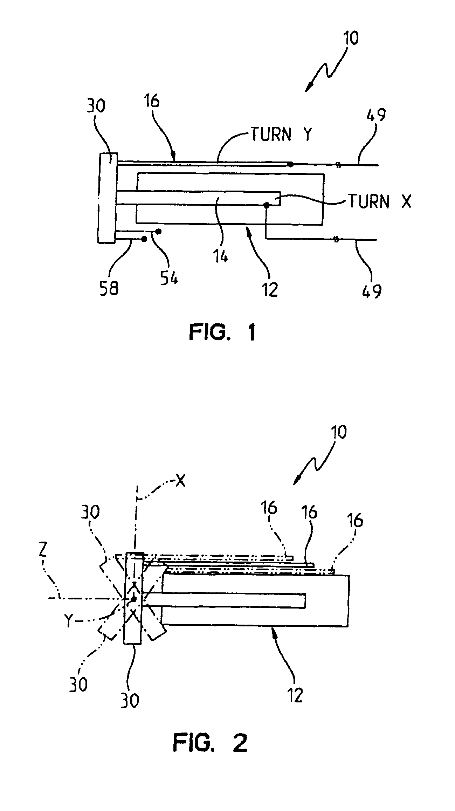

The mechanical driver module of the software communicates with the ultrasound device 400 for determining the movement of, and directing the movement of the device 400 as it moves the elements 412 in their four degrees of freedom (See Fig. 1-3C). The software containing central processing unit 414 is provided for processing the images captured by the device. The CPU 414 that captures the information received by the elements also has the capability of transmitting the information to a remote CPU 420 through traditional means such as the Internet, direct link connection, Network connection, wireless connection and the like.

Turning now to Fig. 12A, the various software modules 415 that are contained within the CPU 414 are shown in a highly schematic manner. The various software modules (or functionalities) are shown in Fig. 12A as comprising four primary functionalities. Depending upon the software architecture used, the various functionalities can be part of a single program, or alternately, can be four separate programs, or some combination thereof.

The functionalities include a general ultrasound device operating functionality 415A. This functionality would be designed primarily to control the operation of the device 400 itself. In addition to this, a device mechanical driver 415B is provided to control the mechanical movement, and mechanically moving and mover components of the device 400. Image processing software 415C is provided for enabling the images taken by the ultrasound device to be processed into an image useable by the user. Additionally, the image processing software 415C should include some means for permitting the user to manipulate the image to better view those items within the image that the user believes to be important.

Further, the image processing software 415C can include a functionality, similar to one that one might find in a program such as Adobe® PhotoShop®. For example, the image processing software 415C, may enable the user to convert the image from black and white to color, change the size of the image, cut and/or crop the image, increase the brightness or the contrast in the image, sharpen the image, or perform one of several other functions to the image to enhance the ability of the image to highlight the pathologies contained within the tissue being shown, or otherwise make the image more useable or more aesthetically pleasing to the user.

A fourth functionality that should be included within the CPU 414's software package is a communications functionality 415D. This communications functionality 415D is provided to permit the CPU 414 to communicate with the remote CPU 420, or other devices that are either within the CPU's 400 network, or are external to the network.

One functionality that the communication software should be able to perform is to enable the CPU 414 and hence the device 400 to transmit information to remote CPU 420.

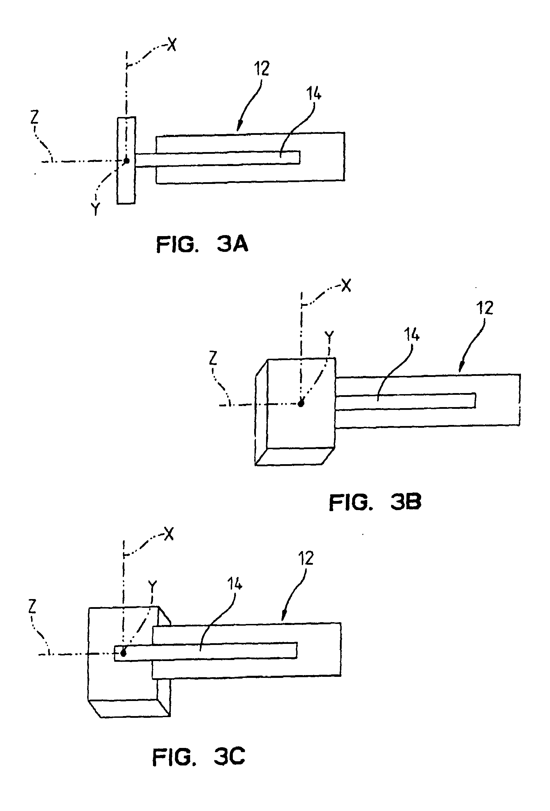

The heart of the ultrasound device 400 resides in the individual ultrasound elements 10 that are shown in Figs. 1-3C. Turning now to Figs. 1-3c, an individual ultrasound element 10 includes a body portion 12 to which a piezoelectric crystal 30 is coupled. The piezoelectric crystal 30 is the component that actually emits the sound of a certain frequency or wave length used with the ultrasound. A pair of motors, including a "Turn X" motor 14 and a "Turn Y" motor 16 are provided for moving the crystal 30. Collectively, motors 14, 16 comprise a piezo motor that cause the crystal 30 to move in four degrees of freedom. The motors 14, 16 comprise a pair of wire-like straps 14 and 16. Piezo strap motor 14 is coupled on one side of the generally square crystal 30 and piezo electric strap motor 16 is coupled to another of the side edges of the crystal 30. Through contraction and expansion of the strap 14, the Piezo crystal 30 can be moved in a direction so that it rotates essentially around axis X, as shown in Figs. 3A-3C. Through the contraction and expansion of strap 16, the piezo crystal 30 can be rotated about an axis Y, shown in Fig. 2. Although the rotation of the crystal is described as being around the X axis and the Y axis, it will be appreciated that the rotation could also be about the Z axis, if that were deemed desirable by the user or manufacturer.

A first wire 49 (Fig. 1) is coupled to the Turn X piezoelectric motor strap 14 mechanical driver that sends a signal to the piezoelectric motor strap 14 to expand or contract, and thereby move to change the orientation of the crystal 30 about the X axis. A second wire 50 is coupled to the second piezoelectric motor strap 16, to cause second strap 16 to move, to cause the crystal 30 to rotate its orientation about axis Y as shown in Fig. 2.

A send wire 54 and a receiver wire 58 are also coupled to the piezoelectric crystal 30. The send wire 54 is used to send information to the piezoelectric crystal 30. The information sent is usually a signal to tell the piezoelectric crystal 30 to send out an acoustical sound signal into the tissue of interest. The receive wire 58 is provided for receiving signals that bounce back to piezoelectric crystal 30 and transmitting these signals ultimately to the CPU 400.

Ultrasound works somewhat similarly to a sound-based radar system where a signal is sent out and is received by the piezoelectric crystal. Through processing performed by appropriate software, by sending and receiving signals and comparing the times, frequencies and types of signals sent and received, one can detect the presence or absence of various tissue types that the signal encounters in its path away from and back to the ultrasound piezoelectric crystal 30. From these signals, and their processing, an image can be constructed from and by the imagine processing software 415D.

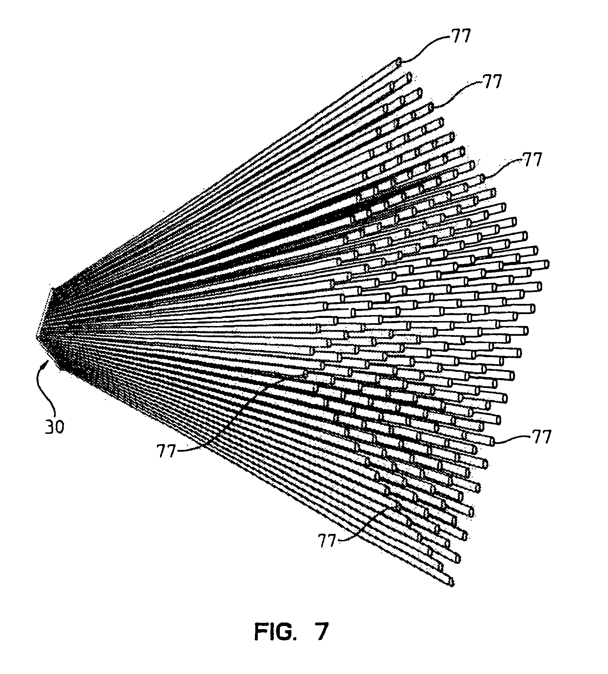

In operation, during the acquisition of data information, the ultrasound element 30 is constantly sending out signals and receiving signals in order to provide the underlying data from which the image is built. In certain living tissues and moving organisms, this image can change, which reflects movement of the tissue, such as the movement incident to the beating of a heart. While this is occurring, the piezoelectric crystal 30 is moved so that it rotates about the X axis and rotates the Y axis. Preferably, it is capable of 20 different positions of rotation along the X axis and 20 different positions of rotation along the Y axis. Turning now to Fig. 7, when the crystal has gone through its entire sequence of rotation around the X axis and then through the entire sequence of rotation around the Y axis, image information is retrieved from an array of data points that are represented by the array of end points 77 shown in Fig. 7.

Because the ultrasound element 30 is capable of movement, the ultrasound element 30 sends information back to the software 415 converts the information it receives from the array of ultrasound elements into a three-dimensional picture of the tissue sample of interest. As such, in order to make this reconstruction, software is needed that is capable of assembling a large number of images together into a coherent three-dimensional image. In order to assemble this data into a three-dimensional image, the computer or image processing software 415C needs: (1) information relating to the image itself; and (2) information that relates to the position of the sensor, so that the position at which the image was "taken" is known by the software 415C. From this information, the software 415C can determine that a particular feature on an image represents a certain point in three-dimensional space in the tissue sample.

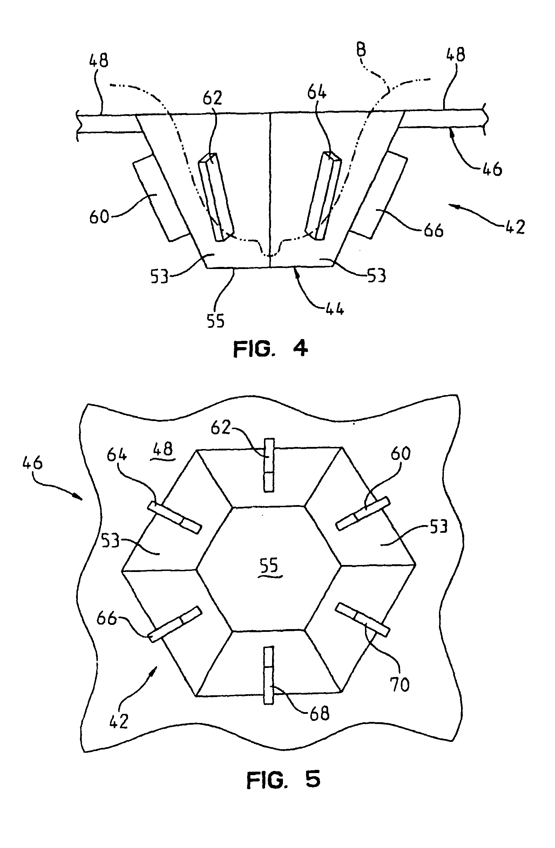

One way to understand this is to look at the breast probe ultrasound device that is shown in Figs. 4 and 5. In Fig. 4, the breast prototype ultrasound device 42 is shown as comprising a generally a six-sided tapered cup 44 that is shown in the top view of Fig. 5. The cup 44 is imbedded in an examination table 46, so that the cup 44 extends below the surface 48 of the table 46. The cup 44 is sized and positioned so that the patient can lay face down, with his/her front side (chest) on the surface 48 of the table 46, with his/her breast B extending into the interior of the cup 44. The cup 44 is preferably water-tight since the interior 50 of the cup is preferably filled with a media as a medium for the ultrasonic waves to travel in during use. Preferably the medium comprises a liquid medium such as water, but can also comprise another type of medium suitable for use with ultrasound, such as a jelly-like medium. Preferably, the water (or other media) is heated up to close to body temperature to improve the comfort level of the test subject.

The cup can be frusto-pyramidal and include six side panels 53 and a hexagonal base panel 55.

Along each of the six side panels 53 of the six-sided cup is disposed a plurality of moveable ultrasound element arrays 60, 62, 64, 66, 68, 70. Each of these arrays 60-70 of moveable ultrasound elements comprises an array of two elements across by 100 elements down (200 total elements). The elements 200 (See Fig. 11) will preferably extend along a significant length of each panel 53, so that the arrays 60-70 can capture images of the entire length of the breast B. This 100 x 2 array may appear similar to the array 198 of elements 200 shown in Fig. 11, but of course, much narrower and much longer than the array 198 of elements 200 shown in Fig. 11. As such, six of these arrays 60, 62, 64, 66, 68 and 70 are contained on the ultrasound device 42.

During the time the ultrasound is being taken, each of the elements 200 on each of these arrays 60-70 is capable of being moved individually and often are moved individually. However, the elements 200 on any particular array, such as the elements on array 66, while being moved individually are preferably all moved synchronously. Turning to Fig. 7, each of the 100 x 2 (200 elements) capable of sending and receiving signals that can be used to capture a plurality of images at a plurality of points so that each element captions a multi-point image similar to the image representation shown in Fig. 7 that results from the elements being moved along the X axis and about the Y axis.

At the same time this is occurring, the same arrays (e.g. 64 and 62) are taking their pictures of the tissue sample of the breast. As can be appreciated, the images taken from the different arrays 60-70 will all be different, because the arrays 60-70 are all "shooting" their images from a different position and at a different angle relative to the tissue of interest. However, since the arrays 60-70 are all shooting the same tissue sample, the images taken can be merged into a single three-dimensional image. This single three-dimensional image can then be viewed in macro to look at the three-dimensional image, or more usually, in a sliced or sectioned configuration to look at the tissue in a particular plane of the tissue.

When the ultrasound device has performed its task and taken all the shots it is going to take with all of the arrays 60-70 and all of the elements being moved, the device will have collected enough data to assemble a three-dimensional view of the tissue sample. This three-dimensional view of the device is assembled by the software that compiles the various images taken by the ultrasound device and correlates them to the tissue areas that they represent so that the software can determine which particular pixels of information correspond to which portion of the three-dimensional tissue sample.

Once this three-dimensional image is assembled, the next step is for the radiologist to review the image in order to make a diagnosis of the tissue from the image. One advantage of the present invention is that the data that is obtained can be manipulated to give the user almost an infinite number of two-dimensional and three-dimensional views of the three-dimensional tissue sample.

As discussed above, the large number of images taken by the large arrays of elements 200 can be compiled by the software 415C so that image information exists for each "point in three-dimensional space" within the tissue sample. Once image information is known for each point in space of the tissue sample, the image of the tissue can then be manipulated to provide the interpreting physician with any one of a virtually unlimited number of "views" of the tissue. The radiologist can then review one or a plurality of different views, as she believes necessary, in order to perform an appropriate review, and make a proper diagnosis.

In some situations, the radiologist will choose to review a three-dimensional view of the body tissue.

In many situations, the three-dimensional view that the device produces is not the only view that contains necessary diagnostic information, but will be viewed in conjunction with other "two dimensional" views. For example, a three-dimensional view of a finger would likely yield a view that appeared be similar to an exterior view of the finger. Similarly, a three dimensional view of a breast sample would likely have an appearance that was generally similar to an exterior view of the breast B.

However, a primary purpose of the ultrasound device is to enable the practitioner to view the interior of the tissue to determine the condition of the interior tissue of the breast or finger or other tissue sample, as that is where the cells of interest, such as the cancer cells are likely to reside. In order to see the interior of the tissue sample, it is preferred to obtain is a thin section view, similar to a thin section view that one might obtain by thin slicing the tissue of the tissue sample. However, because of the plurality of images taken, the present invention enables the practitioner to view a wide array and variety of virtual thin sections of the tumor of interest.

Since the ultrasound device of the present invention is able to obtain information for, in theory, every point in space of the three-dimensional tissue object of interest the data can be manipulated to create an image of any one or more of these pixels of information or any grouping of these pixels. It is believed that the best way to create views that are useful to the practitioner is to create a thin section view, and preferably a plurality of this section view.

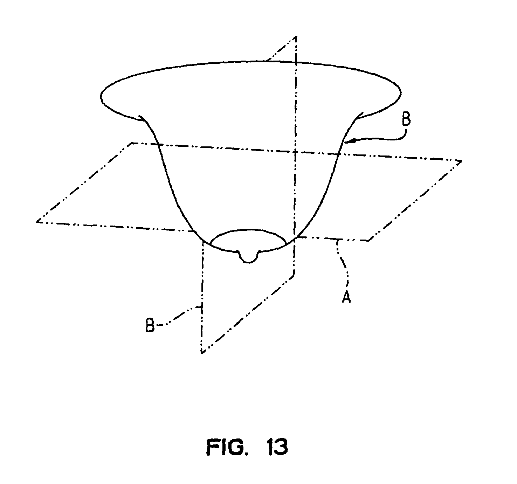

As shown in Fig. 13, the software provides the user with a three-dimensional image, and a pair of virtual cutting planes. The user can place the virtual cutting planes, such as virtual plane A and virtual plane B, in the orientation that the user prefers, and then look at a thin section is taken along that particular cutting plane. As is shown in the drawing, plane A is a plane that is disposed perpendicularly to plane B. It is important to note that since information exists for every pixel, for every point in space within the tissue, information can be gained from a plane at any angle. Although planes A and B are shown as perpendicular virtually and horizontally oriented planes, it will be appreciated that virtual planes A and B could be oblique planes which for example could be oriented 10° or 20° to horizontal; or could be "tilted". One could rotate plane A or B about any axis to achieve another plane, and could by looking at the information contained in a thin section taken along that plane, get the information that one would find by doing a section of the tissue sample along that plane.

Normally, when viewing a new tissue sample, the first thing that the practitioner, would do, is to take a quick "fly by" view of a tissue sample. In practice, this would likely represent, for example, the user taking a "nipple to thoracic bone" view of the breast, by looking at a plurality of slices that were cut through planes similar to plane A shown in Fig. 13, where the first plane that was shown is a plane that is parallel to the chest and that passes through the nipple, and the final plane section is a plane that is parallel to the chest and that is cut through the tissue in a position adjacent to the thoracic bone.

The doctor then uses this "fly by" to find the location of the tumor or cyst (if one exists) or tissue body of interest. Once the practitioner finds the tissue body of interest, he can choose to then look at this tissue body of interest from several different sections, such as section taken along a plane parallel to plane B, and also possibly, a view taken in a plane that is perpendicular to both planes A and B, and possibly other planes. By creating a sufficient amount of thin tissue sample images, wherein the images are taken along a variety of different planes, the user and the software can reconstruct a virtual three-dimensional image of the tumor or cyst of interest. Through this three-dimensional image, the user and diagnostician would be able to gain a better appreciation of the location of the tumor, the shape of the tumor, and the size of the tumor. Through these three items of information, one can gain a better understanding and appreciation of the nature of the tumor and its severity. Additionally, if temporally spaced sequential ultrasounds were taken of the tissue body of interest over time, one would be able to measure the growth and determine the growth rate (or lack of growth) of the tumor to better determine the aggressiveness of the growth of the tumor.

The biopsy procedure is also enhanced by the present invention's capability of precisely locating the tumor in three-dimensional space. A biopsy is normally conducted by a very thin needle being inserted into the tissue of interest, and the tissue of interest being sucked out or otherwise retracted by the biopsy needle. The removed biopsy tissue is then tested through various diagnostic procedures to determine the nature of the tissue removed. Often, in the case of a tumor, the determination involves determining whether the tumor is benign or cancerous.

One difficulty that occurs when performing a biopsy is determining the precise location of the tissue of interest. By using the present invention, the practitioner has a better image of the tumorous tissue, and better location data relating to the three-dimensional positioning of tumorous tissue, since the present invention provides the practitioner with a three-dimensional map of both the "organ" (e.g. breast, pancreas, etc.) and also the potentially tumorous tissue within the "organ".

Additionally, if the ultrasound device of the present invention is used simultaneously with the insertion of the biopsy needle, the ultrasound image information provided by the present invention would be real-time information. Since the information is delivered in real time, one can view the ultrasound image of a tissue of interest as the biopsy needle is inserted into the organ, to ensure that the biopsy needle becomes inserted within the tumorous tissue to insure that the biopsy needle is in fact removing the desired tumorous tissue, rather than tissue outside of the body of interest.

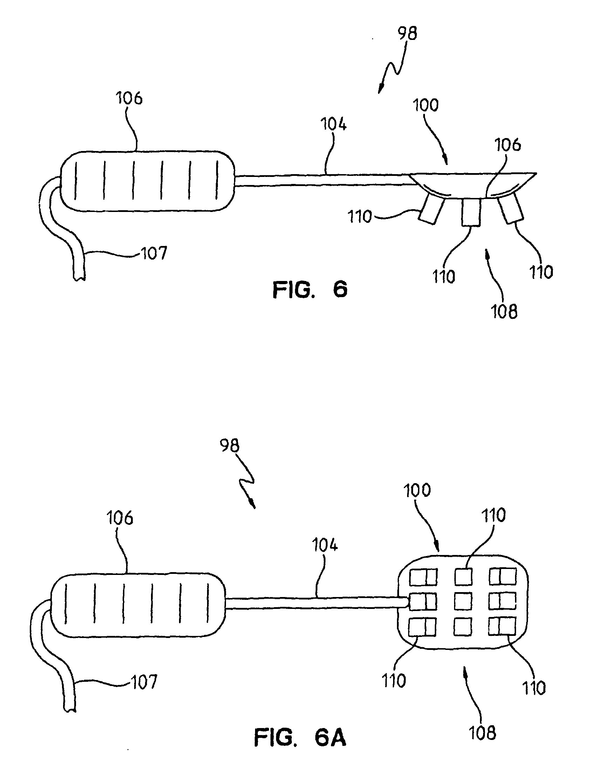

Turning now to Fig. 6 and 6A, another type of probe 90 is shown. Probe 98 is the type that one might use for a prostate exam. Since the prostate probe 98 is being used internally and in this case, intra-rectally, size and shape constraints imposed by the need to insert the probe intra-rectally create significant design restraints that impact the size and shape of the probe. As one can imagine, a probe as large as one might use on the breast can not comfortably be used when the probe is being inserted internally.

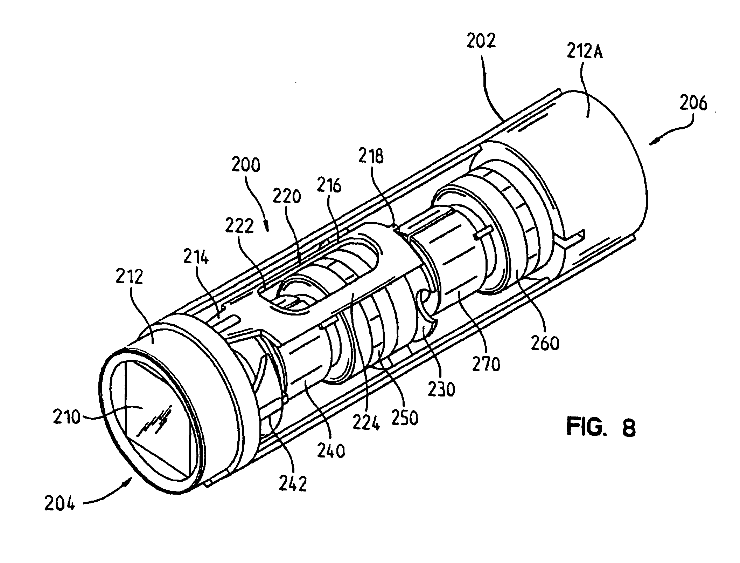

It is envisioned that such a probe 98 will include head 100, a shaft 104 both of which are insertable in the rectum. The probe 98 also includes a handle 106 that is disposed externally during an examination. The practitioner uses the handle 106 to manipulate the probe 98 and more importantly, the probe head 100 within the colon. The probe head 100 preferably has a concave shaped wall engaging surface 106 that may be shaped similarly to a table spoon head. This concave surface 106 is placeable against the wall of the rectum adjacent to the prostate, so that the prostate can rest within the concave scoop surface 106. On the backside of the concave surface 106 is the array 108 of ultrasound elements 110, which in this case is probably a 3 x 3 array of ultrasound elements 110. Ultrasound elements 110 are preferably miniaturized versions of the elements shown in Figs. 8-10. A communication chord 107 or radio transmitter (not shown) is provided to enable the probe 98 to communicate with CPU 400.

In order to operate this device, the ultrasound probe 98 is coupled to the CPU 400, and both are powered up. The probe 98 is then inserted through the patient's anus and into the interior of the patient's colon, with handle 106 being held externally and used to manipulate the probe head 100 into a position wherein the concave scoop surface 106 is positioned against a wall of the colon at a position adjacent to the patient's prostate. When the probe head 100 is positioned properly, the driver software 415B (Fig. 12A) will then be instructed to cause the 3 x 3 array 108 of ultrasound elements 110 to take images while the elements 110 move along the their X axis and their Y axis. The images then taken will be transmitted to the computer 400 where they will be processed by the image processing software 415C so that a three-dimensional view of the prostate will be constructed. The three-dimensional view that is obtained from the array 108 of ultrasound elements 110 of the probe 98 will be constructed by a method analogous to the manner in which a three-dimensional image is constructed by your eyes through this through stereoscopic vision. When an image is "shot" from two different sensors that are spaced far enough apart, the images are merged three-dimensional information about the object of interest can be obtained.

Once the ultrasonically captures a three dimensional image of the prostate, the image will then be analyzed by the urologist (or pathologist or radiologist) in much the way that the breast was analyzed. In particular, the imaging software 415C will be employed to create a plurality of thin section images constructed along a variety of different planes to thereby provide a plurality of virtual thin sections taken from a plurality of angles. As also with the breast procedure described above, the imaging software can be employed to create a three dimensional image of the prostate by having the cut plates, so that the radiologist can view a variety of virtual thin section images or views of the prostate.

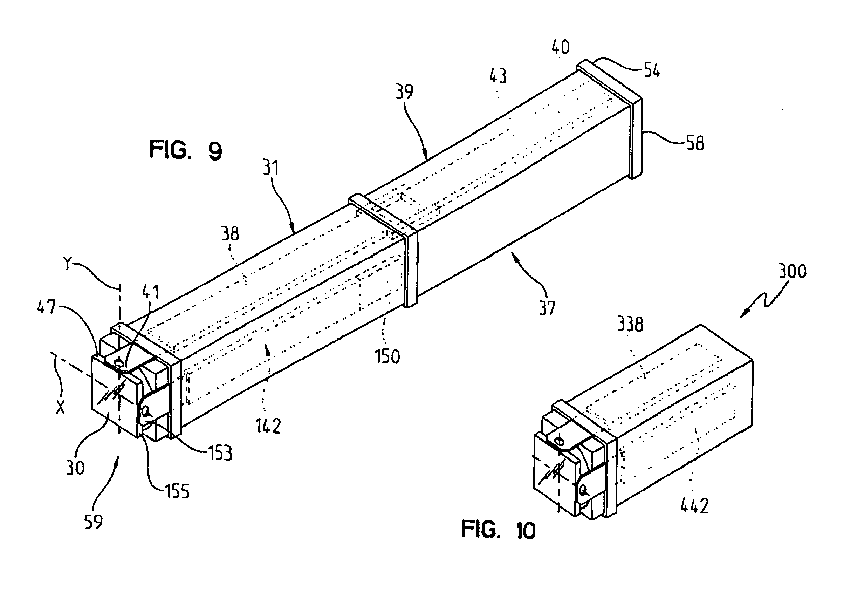

Turning now to Figs. 8-10, the construction of three alternate embodiment elements 200, 37, 300 is discussed. The embodiments include cylindrical element 200 shown in Fig. 8; the long rectangular cuboid element 37 shown in Fig. 9; and the short rectangular cuboid element 300 shown in Fig. 10.

Turning first to Fig. 8, the cylindrical element 200 includes a piezoelectric crystal 210, which comprise a 4 mm x 4 mm element 210 that is disposed at one end of the element 200. The element 200 is shown as having a generally cylindrical configuration and includes a body portion comprising an exterior housing 202, and a first or "head" end 204, and a second or "base" end 206. The piezoelectric crystal 210 is disposed at the head end 204. The housing 202 includes a circular collar portion 212 disposed adjacent to the head end 204, whose purpose is to help couple the piezoelectric element 200 onto the array base 388 (Fig. 11).

It will be appreciated that although the housing 202, generally encloses all of the internal components, the view shown in Fig. 8 comprises a cut-away view so that the internal components can be explained and understood by the reader. The housing 202 generally extends between the collar 212 and the second end 206. It will be noted that the base end portion 212A of the housing is configured into a base for receiving the wires for driving the piezoelectric element 210, and for serving as a base onto which the internal components can be mounted.

A piezoelectric motor is provided for moving the piezoelectric crystal element 210 along its X and Y axis in four degrees of freedom. The motor assembly includes a longitudinal member 220 and a circular motor member 230. The longitudinal member 220 includes a proximal end 218 disposed relatively closer to the base end 206, and a distal end 214, disposed relatively closer to the head end 204. The distal end 214 is coupled onto the sides of the piezoelectric crystal 210, in a manner generally similar to that shown in connection with Figs. 1-3C.

The longitudinal member 220 also includes an intermediate portion, that consists of two axially extending metal bands 222, 224. The axially extending bands 222, 224 are capable of expanding or contracting in an axial direction. Through this axial expansion and contraction, the axially extending band 222, 224 can move the piezoelectric crystal 210.

The proximal end 218 of the longitudinal member 220 includes a coupling for coupling the proximal end 218 of the longitudinal member 220 to the circular member 230. The circular member 230 is disposed in a generally radially extending plane that is generally perpendicular to the axis of the longitudinal member 220. The circular member 230 extends radially outwardly, and has an axis that extends generally co-linearly with the axis of the element 200.

The element 200 also includes a pair of lateral stabilizers 250, 260. The lateral stabilizers 250, 260 do not necessarily perform any role within the transmission and reception of ultrasound waves, or the movement of the piezoelectric crystal 210. Rather, they help to stabilize the device and provide couplers and attachments points for maintaining the internal components within the element 200.

A central shaft element 270 is operatively coupled to a Y axis mover 240. The Y axis mover 240 has segments 242 that are coupled to the side of the piezoelectric crystal 210. Segments 242 move the piezoelectric crystal 210 about its Y axis, as compared to member 214 that moves the piezoelectric crystal 210 about its X axis.

Wires (not shown) are provided for providing electrical signals to the various components, such as the band 220 and the Y mover 240, to cause these bands 220, 240 to expand and contract, and thereby move the piezoelectric crystal 210.

Referring to Fig. 9, a rectangular cuboid-shaped ultrasound element 37 includes a housing 39 that includes a base end 58, and a head end 59. A moveable piezoelectric crystal 30 is disposed at the head end, similar to piezoelectric crystal 210 of Fig. 8.

An X axis moveable band is provided that includes a first segment 38 and a second segment 43.

The first mover 31 includes a first segment 38 and a second segment 43. The first end 40 of the first mover 31 is coupled to the body portion 40, and the second end 41 of the mover 31 is coupled to the side edge portion 47 of the piezoelectric crystal 30. Similarly, the first end 150 of the second mover 142 is coupled to the body portion 39, and the second end 153 is coupled to a second side edge portion 155 of the piezoelectric crystal 30.

A current is applied to second segment 43 that causes the second segment 43 to either expand or contract. First segment 38 does not necessarily expand or contract, but rather serves as an extender that moves in response to the expansion or contraction of second segment 43. Segment 38 of the X movable band is coupled to the side surface 47 of the piezoelectric crystal 30 to move the piezoelectric crystal about the X axis.

Similarly, a Y moveable band 142 is provided that is coupled at its 142 distal end to a side edge of the piezoelectric crystal 30. A signal is applied to band 142 to enable it to expand or contract, to thereby move the piezoelectric crystal about a Y axis. The proximal (first) end 150 of the moveable band 142 is coupled to either another band extender (not shown) or to a base member. Additionally, a base end member 40 is disposed at the proximal end of the element 37.

A shortened rectangular cuboid element 300 is shown in Fig. 10. Shortened element 300 also includes an X moveable band 338, and a Y moveable band 342. Generally, shortened rectangular cuboid element 300 is constructed similarly to, but shorter than the elongated rectangular cuboid element 37 shown in Fig. 9.

Finally, turning now to Fig. 11, an array of elements 200 is shown. The array of elements 200 are coupled onto a frame 388. Frame 388 is generally stationarily positioned. Each of the elements 200 is individually moveable with respect to the frame 388. Preferably, the elements 200 move simultaneously and synchronously, because such movement helps to reduce the processing burden placed upon the central processing unit 400 that is processing the images captured by the piezoelectric crystals 390 of the array 198 of element 200.

In the foregoing description relating to the elements 200, it will be appreciated that generally, the barrel portions of the elements 200 remain fixedly positioned with respect to the frame 388, with the only moving member of the elements 200 being the piezoelectric crystals 390, that can move along X and Y axes in four degrees of freedom by the piezoelectric motor. It will be noted that the elements 200 shown in Fig. 11 are constructed similarly to element 200 shown in Fig. 8. However, it will also be appreciated that the elements 37, 300 shown in Figs. 9 and 10, respectively, could be substituted for the generally cylindrical elements 200 shown in Fig. 11.

Although the invention has been described with reference to certain preferred embodiments, it will be appreciated that variations and modifications exist within the scope of the claimed invention.

## Claims

1. An ultrasound device (198) for diagnosing pathologies in a tissue of interest comprising a platform member (388); and an array of at least two ultrasonic elements (200) coupled to the platform member (388), each of the at least two elements including a body portion (39, 202), a piezoelectric crystal member (30, 210) having a first axis (X) and a second axis (Y), a first mover (31) capable of moving the piezoelectric crystal member about the first axis (X) of the piezoelectric crystal member (30), and a controller (400) for causing the first mover (31) to move the piezoelectric crystal member (30) about the first axis (X) for enabling the ultrasound device (198) to capture a plurality of images at different positions of rotation about the first axis (X), **characterized in that**
each of the at least two elements includes a second mover (142) capable of moving the piezoelectric crystal member (30) about the second axis (Y) of the piezoelectric crystal member (30); and
the controller (400) is also for causing the second mover (142) to move the piezoelectric crystal member (30) about the second axis (Y) for enabling the ultrasound device (198) to capture a plurality of images at different positions of rotation about the second axis (Y).

2. The ultrasound device ofclaim 1, wherein the first (X) and second (Y) axes of the piezoelectric crystal member (30) comprise respectively an X-axis and a Y-axis, and wherein the first mover (31) is capable of moving the piezoelectric crystal member (30) about the X-axis, and the second mover (142) is capable of moving the piezoelectric crystal member (30) about the Y-axis, further comprising imaging software (415C) for correlating the plurality of images to produce at least one of a plurality of thin section images and a three-dimensional image.

3. The ultrasound device of claim 2, wherein the plurality of the section images produced comprise at least one of a plurality of thin section images showing parallel planes of the tissue of interest, a plurality of views of the tissue of interest showing different aspects of the tissue of interest, and a three-dimensional view of a tissue of interest within the tissue sample.

4. The ultrasound device of any preceding claim, wherein the first mover (31) comprises a band comprised of a material capable of expanding and contracting under the influence of energy applied to the band (31), wherein the expansion and contraction of the material of the band (31) moves the piezoelectric crystal member (30) about the first axis (X).

5. The ultrasound device of claim 4, wherein the band (31) comprises a material band having a first end (40) coupled to the body portion (39) of the ultrasonic element (37) and a second end (41) coupled to a first edge portion (47) of the piezoelectric crystal member (30).

6. The ultrasound device of any preceding claim, wherein the second mover (142) comprises a metal band (142) capable of expanding and contracting under the influence of the application of energy to the band (142), where the expansion and contraction of the metal of the band (142) moves the piezoelectric crystal member (30) about its second axis (Y), and wherein the band (142) includes a first end (150) coupled to the body portion (39) of the ultrasonic element (37) and a second end (153) coupled to a second edge portion (155) of the piezoelectric crystal member (30).

7. The ultrasound device of any preceding claim, further comprising a tissue receiving member (44), wherein the platform member (64) is fixedly coupled to the tissue receiving member (44) and the body portions (39) of the at least two elements (37) are fixedly coupled to the platform member (64), and wherein the piezoelectric crystal members (30) are movable relative to the platform (64) and body portion (39).

8. The ultrasound device of claim 7, wherein the tissue receiving member (44) comprises a cup member (44) capable of receiving a media and the tissue of interest, the cup (44) comprising the platform member (60-70) to which the array of at least two ultrasonic elements (37) are coupled, and the array of at least two of ultrasonic elements (37) comprises at least four arrays (60-70) of at least two ultrasonic elements (37) circumferentially arrayed around the cup (44) to enable the ultrasonic elements (37) to capture images fully around an axis of the tissue of interest.

9. The ultrasound device of claims 7 or 8, wherein the tissue receiving member comprises a head portion (100) of a hand held instrument (98) that also includes a handle portion (106), the head portion (100) comprising the platform to which the array of at least two ultrasonic elements (110) are attached.

10. The ultrasound device of claim 9, wherein the head portion (106) is sized and configured for insertion into a mammalian orifice for permitting the head portion (106) to be positioned internally within the orifice and adjacent to an internally disposed tissue of interest.

## Patentansprüche

1. Ultraschallvorrichtung (198) zur Diagnose von Krankheiten in einem interessierenden Gewebe, umfassend ein Plattformteil (388); und eine Anordnung von mindestens zwei an dem Plattformteil (388) gekoppelten Ultraschallelementen (200), wobei jedes der zwei Elemente ein Körperteil (39, 202), ein piezoelektrisches Kristallteil (30, 210) mit einer ersten Achse (X) und einer zweiten Achse (Y), einen zur Bewegung des piezoelektrischen Kristallteils um die erste Achse (X) des piezoelektrischen Kristallteils (30) geeigneten ersten Antrieb (31), und eine Steuervorrichtung (400), die bewirkt, dass der erste Antrieb (31) das piezoelektrische Kristallteil (30) um die erste Achse (X) bewegt, um der Ultraschallvorrichtung (198) die Aufnahme einer Vielzahl von Abbildungen in verschiedenen Rotationspositionen um die erste Achse (X) zu ermöglichen, beinhaltet, **dadurch gekennzeichnet, dass**
jedes der mindestens zwei Elemente einen zweiten Antrieb (142) beinhaltet, der zur Bewegung des piezoelektrischen Kristallteils (30) um die zweite Achse (Y) des piezoelektrischen Kristallteils (30) geeignet ist, und dass die Steuervorrichtung (400) auch den zweiten Antrieb (142) veranlasst, das piezoelektrische Kristallteil (30) um die zweite Achse (Y) zu bewegen, um der Ultraschallvorrichtung (198) die Aufnahme einer Vielzahl von Abbildungen in verschiedenen Rotationspositionen um die zweite Achse (Y) zu ermöglichen.

2. Ultraschallvorrichtung nach Anspruch 1, wobei die erste (X) und zweite (Y) Achse des piezoelektrischen Kristallteils (30) jeweils eine X-Achse und eine Y-Achse umfassen, und wobei der erste Antrieb (31) zur Bewegung des piezoelektrischen Kristallteils (30) um die X-Achse geeignet ist, und der zweite Antrieb (142) zur Bewegung des piezoelektrischen Kristallteils (30) um die Y-Achse geeignet ist, wobei die Ultraschallvorrichtung weiter eine Bildgebungssoftware (415C) zur Korrelation der Vielzahl von Abbildungen für die Erzeugung zumindest eines von einer Vielzahl von dünnen Schnittbildern und einer dreidimensionalen Abbildung umfasst.

3. Ultraschallvorrichtung nach Anspruch 2, wobei die Vielzahl der hergestellten Schnittbilder zumindest eines aus einer Vielzahl von dünnen Schnittbildern, die parallele Ebenen des interessierenden Gewebes zeigen, eine Vielzahl von Ansichten des interessierenden Gewebes, die verschiedene Aspekte des interessierenden Gewebes zeigen, und eine dreidimensionale Ansicht des interessierenden Gewebes innerhalb der Gewebeprobe umfasst.

4. Ultraschallvorrichtung nach einem der vorhergehenden Ansprüche, wobei der erste Antrieb (31) ein Band umfasst, das aus einem unter Einfluss von auf das Band (31) angewandter Energie streckbaren und zusammenziehbaren Material besteht, wobei das Strecken und das Zusammenziehen des Materials des Bands (31) das piezoelektrische Kristallteil (30) um eine erste Achse (X) bewegt.

5. Ultraschallvorrichtung nach Anspruch 4, wobei das Band (31) ein Materialband umfasst, dass ein an das Körperteil (39) des Ultraschallelements (37) gekoppeltes erstes Ende (40) und ein an einen ersten Kantenbereich (47) des piezoelektrischen Kristallteils (30) gekoppeltes zweites Ende (41) aufweist.

6. Ultraschallvorrichtung nach einem der vorhergehenden Ansprüche, wobei der zweite Antrieb (142) ein unter dem Einfluss der Anwendung von Energie auf das Band (142) streckbares und zusammenziehbares Metallband (142) umfasst, wobei das Strecken und das Zusammenziehen des Metalls des Bands (142) das piezoelektrische Kristallteil (30) um seine zweite Achse (Y) bewegt, und wobei das Band (142) ein an das Körperteil (39) des Ultraschallelements (37) gekoppeltes erstes Ende (150) und ein an ein zweites Kantenteil (155) des piezoelektrischen Kristallteils (30) gekoppeltes zweites Ende (153) beinhaltet.

7. Ultraschallvorrichtung nach einem der vorhergehenden Ansprüche, weiter umfassend ein Gewebeaufnahmeteil (44), wobei das Plattformteil (64) fest an das Gewebeaufnahmeteil (44) gekoppelt ist und die Körperteile (39) der zumindest zwei Elemente (37) fest an das Plattformteil (64) gekoppelt sind, und wobei die piezoelektrischen Kristallteile (30) relativ zu dem Plattform- (64) und Körperteil (39) beweglich sind.

8. Ultraschallvorrichtung nach Anspruch 7, wobei das Gewebeaufnahmeteil (44) ein zur Aufnahme von Medien und dem Gewebe geeignetes Becherteil (44) umfasst, der Becher (44) umfasst das Plattformteil (60-70), an dem die Anordnung von mindestens zwei Ultraschallelementen (37) gekoppelt ist, und die Anordnung von mindestens zwei Ultraschallelementen (37) umfasst mindestens vier Anordnungen (60-70) von mindestens zwei um den Becher (44) umlaufend angeordneten Ultraschallelementen (37), um den Ultraschallelementen (37) die Aufnahme von Abbildungen vollständig um eine Achse des interessierenden Gewebes zu ermöglichen.

9. Ultraschallvorrichtung nach Anspruch 7 oder 8, wobei der Gewebeaufnahmeteil ein Kopfteil (100) eines Handinstruments (98) umfasst, das auch einen Bedienbereich (106) beinhaltet, wobei das Kopfteil (100) die Plattform, an der die Anordnung von zumindest zwei Ultraschallelementen (110) angebracht ist, umfasst.

10. Ultraschallvorrichtung nach Anspruch 9, wobei das Kopfteil (106) zur Einbringung in Körperöffnungen von Säugetieren ausgelegt und konfiguriert ist, um dem Kopfteil (106) die Positionierung innerhalb der Körperöffnung und gegenüber einem innen angeordneten interessierenden Gewebe zu ermöglichen.

## Revendications

1. Dispositif à ultrasons (198) pour le diagnostic de pathologies dans un tissu d'intérêt comprenant un élément plateforme (388) ; et un réseau d'au moins deux éléments ultrasoniques (200) accouplé à l'élément plateforme (388), chacun desdits au moins deux éléments comprenant une partie corps (39, 202), un élément cristal piézoélectrique (30, 210) ayant un premier axe (X) et un second axe (Y), un premier dispositif de déplacement (31) capable de déplacer l'élément cristal piézoélectrique autour du premier axe (X) de l'élément cristal piézoélectrique (30), et un dispositif de commande (400) destiné à entraîner le déplacement par le premier dispositif de déplacement (31) de l'élément cristal piézoélectrique (30) autour du premier axe (X) pour permettre au dispositif à ultrasons (198) de capturer une pluralité d'images à différentes positions de rotation autour du premier axe (X), **caractérisé en ce que**
chacun des au moins deux éléments comprend un second dispositif de déplacement (142) capable de déplacer l'élément cristal piézoélectrique (30) autour du second axe (Y) de l'élément cristal piézoélectrique (30) ; et
le dispositif de commande (400) est également destiné à entraîner le déplacement par le second dispositif de déplacement (142) de l'élément cristal piézoélectrique (30) autour du second axe (Y) pour permettre au dispositif à ultrasons (198) de capturer une pluralité d'images à différentes positions de rotation autour du second axe (Y).

2. Dispositif à ultrasons selon la revendication 1, dans lequel les premier (X) et second (Y) axes de l'élément cristal piézoélectrique (30) comprennent respectivement un axe X et un axe Y, et dans lequel le premier dispositif de déplacement (31) est capable de déplacer l'élément cristal piézoélectrique (30) autour de l'axe X, et le second dispositif de déplacement (142) est capable de déplacer l'élément cristal piézoélectrique (30) autour de l'axe Y, comprenant en outre un logiciel d'imagerie (415C) destiné à corréler la pluralité d'images pour produire au moins l'une d'une pluralité d'images de section mince et d'une image en trois dimensions.

3. Dispositif à ultrasons selon la revendication 2, dans lequel la pluralité d'images de section produites comprend au moins l'une d'une pluralité d'images de section mince présentant des plans parallèles du tissu d'intérêt, d'une pluralité de vues du tissu d'intérêt présentant différents aspects du tissu d'intérêt, et d'une vue en trois dimensions d'un tissu d'intérêt à l'intérieur de l'échantillon de tissu.

4. Dispositif à ultrasons selon l'une quelconque des revendications précédentes, dans lequel le premier dispositif de déplacement (31) comprend une bande composée d'un matériau capable de se dilater et de se contracter sous l'influence d'énergie appliquée à la bande (31), dans lequel la dilatation et la contraction du matériau de la bande (31) font se déplacer l'élément cristal piézoélectrique (30) autour du premier axe (X).

5. Dispositif à ultrasons selon la revendication 4, dans lequel la bande (31) comprend une bande de matériau ayant une première extrémité (40) accouplée à la partie corps (39) de l'élément ultrasonique (37) et une seconde extrémité (41) accouplée à une première partie bord (47) de l'élément cristal piézoélectrique (30).

6. Dispositif à ultrasons selon l'une quelconque des revendications précédentes, dans lequel le second dispositif de déplacement (142) comprend une bande de métal (142) capable de se dilater et de se contracter sous l'influence de l'application d'énergie à la bande (142), où la dilatation et la contraction du métal de la bande (142) font se déplacer l'élément cristal piézoélectrique (30) autour de son second axe (Y), et dans lequel la bande (142) comprend une première extrémité (150) accouplée à la partie corps (39) de l'élément ultrasonique (37) et une seconde extrémité (153) accouplée à une seconde partie bord (155) de l'élément cristal piézoélectrique (30).

7. Dispositif à ultrasons selon l'une quelconque des revendications précédentes, comprenant en outre un élément de réception du tissu (44), dans lequel l'élément plateforme (64) est fixement accouplé à l'élément de réception de tissu (44) et dans lequel les parties corps (39) desdits au moins deux éléments (37) sont fixement accouplées à l'élément plateforme (64), et dans lequel les éléments cristaux piézoélectriques (30) sont amovibles relativement à la plateforme (64) et à la partie corps (39).

8. Dispositif à ultrasons selon la revendication 7, dans lequel l'élément de réception de tissu (44) comprend un élément cupule (44) capable de recevoir un milieu et le tissu d'intérêt, la cupule (44) comprenant l'élément plateforme (60-70) auquel le réseau d'au moins deux éléments ultrasoniques (37) est accouplé, et le réseau d'au moins deux éléments ultrasoniques (37) comprend au moins quatre réseaux (60-70) d'au moins deux éléments ultrasoniques (37) arrangés de manière circonférentielle autour de la cupule (44) pour permettre aux éléments ultrasoniques (37) de capturer des images autour de la totalité d'un axe du tissu d'intérêt.

9. Dispositif à ultrasons selon la revendication 7 ou 8, dans lequel l'élément de réception de tissu comprend une partie tête (100) d'un instrument manuel (98) qui comprend également une partie poignée (106), la partie tête (100) comprenant la plateforme à laquelle le réseau d'au moins deux éléments ultrasoniques (110) est fixé.

10. Dispositif à ultrasons selon la revendication 9, dans lequel la partie tête (106) a une taille et une configuration permettant son insertion dans un orifice d'un mammifère pour permettre le positionnement de la partie tête (106) de manière interne à l'intérieur de l'orifice et adjacente à un tissu d'intérêt disposé intérieurement.
